# EUROPEAN PATENT APPLICATION

(11) **EP 4 455 280 A1**
(43) Date of publication of application: **30.10.2024**
(21) Application number: 22910050.8
(22) Date of filing: 21.12.2022
(51) Int. Cl.: C12N 9/18, C12N 1/15, C11B 3/00, C12R 1/685

(54) **MUTATED LYSOPHOSPHOLIPASE, AND MUTATED ASPERGILLUS NIGER STRAIN FOR EXPRESSING LYSOPHOSPHOLIPASE**

(30) Priority: 23.12.2021 CN 202111586420; 24.12.2021 CN 202111598184
(71) Applicant: Wilmar (shanghai) Biotechnology Research & Development Center Co., Ltd., Shanghai 200137 (CN)
(72) Inventor: XUAN, Yaoji, Shanghai 200137 (CN); WU, Wei, Shanghai 200137 (CN); SUN, Jianan, Shanghai 200137 (CN); LIU, Guorui, Shanghai 200137 (CN); DAI, Xiaojun, Shanghai 200137 (CN); NIU, Qiwen, Shanghai 200137 (CN); WANG, Shengnan, Shanghai 200137 (CN)
(74) Representative: Patentanwälte Ruff, Wilhelm, Beier, Dauster & Partner mbB
(86) International application number: PCT/CN2022/140539
(87) International publication number: WO 2023/116738

(57) **Abstract**

Provided is a mutated lysophospholipase. Further provided are a gene encoding the lysophospholipase, and a vector and a host cell comprising the gene. In addition, further provided is a use of the lysophospholipase. The present invention also provides a mutated *Aspergillus niger* strain and a use thereof. Specifically, the present invention provides a mutated *Aspergillus niger* strain, which is an orotate phosphoribosyltransferase auxotrophic strain, and has improved production capacity of endogenous enzymes, preferably lysophospholipase, with respect to an unmutated strain. The present invention further provides uses of the strain. For example, the strain can be used for expressing the mutated lysophospholipase of the present invention.

## Description

### Field

The present invention relates to the field of biotechnology, and more specifically relates to a mutated lysophospholipase. The present invention also relates to a gene encoding said enzyme, as well as a vector and a host cell comprising said gene. Furthermore, the use of said enzyme is also related to.

The present application also relates to a mutated *Aspergillus niger* strain that can be used to express the mutated lysophospholipase of the invention. The invention also relates to a method for producing exogenous proteins, in particular the lysophospholipase of the invention using the strain.

### Background

*Aspergillus niger* is a common species of fungi belonging to the subphylum Ascomycota, the family Moniliacea, and the genus Aspergillus. It is also the most common filamentous fungus in nature and is widely distributed in plant products, food and soil. The conidial heads thereof are dark brown radial, globular apical capsule. The conidiophores thereof vary in length, and the mycelia thereof are well developed and multi-branched. *Aspergillus niger* can grow rapidly by degrading organic matter in nature and absorbing nutrients therein. *Aspergillus niger* is classified as GRAS (Generally Regards As Safe) by the U.S. Food and Drug Administration (FDA) and recognized by the World Health Organization. It is a very commonly used species among filamentous fungi. *Aspergillus niger* has become an important strain for industrial production because of its ability to produce enzyme preparations and organic acids.

*Aspergillus niger* has efficient protein secretion and expression capabilities. The foreign proteins expressed by *Aspergillus niger* have the characteristics such as large expression amount, high extracellular secretion rate, and protein molecular folding and modification system close to higher eukaryotic cells, and the expressed foreign proteins having natural activities. In addition, *Aspergillus niger* can also perform various post-translational processes, such as glycosylation modification, protease cleavage and disulfide bond formation. Therefore, the use of *Aspergillus niger* as an expression strain to express homologous and heterologous proteins has attracted increasing attention. Commercial enzyme preparations currently produced using *Aspergillus niger* include amylase, glucose oxidase, catalase, cellulase, pectinase, protease, phytase and xylanase etc. used in food, detergent, textile and paper industries. The heterologous proteins expressed by it include lysozyme, interleukin-6, human lactoferrin, bovine chymosin, thaumatin, lipase, etc.

Enzyme preparations used in food in China must comply with the National Standard of the People's Republic of China GB2760 - National Food Standard\Food Additive Usage Standard. Among them, the heterologously expressed lysophospholipases for food (derived from *Aspergillus niger*) that meet the regulations can only be heterologously expressed by *Aspergillus niger.*

Two lipases have been reported in *Aspergillus niger,* named as lipaseA and lipaseB, or lipase1 and lipase2. Among them, lipaseB has special properties. Zhu Shu-sen has cloned and expressed lipaseB from *Aspergillus niger* A733 and has found that the optimal temperature of lipaseB is 15°C, the optimal pH is 3.5-4.0, and it cannot tolerate temperatures above 40°C. The enzyme can hydrolyze substrates with chain lengths of pNPC4-pNPC18, wherein pNPC12 is the optimal substrate. However, the specific enzyme activity of lipB is extremely low, and the specific enzyme activity of purified lipB is only 6.8U/mg.

Jiangke Yang et al. have cloned and expressed lipas2 from *Aspergillus niger* CICC 4009. Although this lipase2 is highly homologous to the lipaseB cloned by Zhu Shu-sen, with only two amino acid differences, there are certain differences in properties. The optimal substrates are pNPC8 and pNPC10, the optimal pH is less than 6.5, the optimal temperature is 50°C, and it cannot tolerate temperatures above 40°C.

In summary, liapse2 or lipaseB derived from *Aspergillus niger* is not very practical. First of all, the specific enzyme activity is extremely low and the temperature tolerance is not high. The most suitable substrates are triglycerides of short-chain fatty acids. This enzyme is not as good as the more widely used lipases TL and RML, which have specific enzyme activity of 12000 or 8000U/mg. TL can tolerate temperature of 60°C for 20 hours without inactivation, and RML can hydrolyze triglycerides of various long-chain fatty acids.

In the invention CN2021107079194, the inventor used the gene sequence of lipaseB of CBS513.88 from *Aspergillus niger* GIM 3.24 (AN02) to design primers and cloned the LPL gene AN02-LPL of the above-mentioned *Aspergillus niger* strain, and found that AN02-LPL had a very high phospholipase A1 activity and lysophospholipase activity, and could be used for oil degumming. Without adding alkali, the phosphorus content of crude oil could be reduced to 5ppm. It can be used for enzymatic degumming and can reduce the formation of soap during degumming because there is no need to add alkali. In addition, because AN02-LPL has extremely high lysophospholipase activity, it can be used in combination with phospholipase A2 to use soybean phospholipase as raw material to prepare glycerol phospholipase (GPC), which has brain-building and anti-aging effects and is used in medicine and in health care products.

In CN2021107079194, the inventor obtained the LPL encoded by the AN02 mutant picAN02m1 by random mutation. The mutation sites are L86I, G187D, E209K, and A245D. The thermal stability is 3.2 times, 31 times and 28.5 times higher than that of the wild type AN02-LPL under the conditions of pH 5.6, 6.0 and 6.6 respectively.

There is still a need in the art to obtain lysophospholipases with higher specific enzymatic activities. Moreover, there is still a need in the art for *Aspergillus niger* strains that can efficiently express various proteins, especially lysophospholipases for food.

### Summary of the invention

Based on previous work and the mutated lysophospholipase developed in invention CN2021107079194, the inventor developed new mutants. Specifically, after mutating the threonine at position 255 of LPL to tryptophan or phenylalanine on the basis of picAN02m1, the specific enzyme activities of the lysophospholipases were increased from 12746 U/mg and 13473U/mg of LPLs encoded by picAN02 and picAN02m1 to 30224U/mg and 18973U/mg respectively. This further improves the efficiency of using the lysophospholipase to produce glycerol phosphorylcholine (GPC).

In addition, the present invention used *Aspergillus niger* CICC2243 as the starting strain to construct an orotate phosphoribosyltransferase auxotrophic strain (pyrE-) strain, and then through ARTP mutagenesis, a strain was screened which had a significantly larger sedimentation circle compared with the starting strain on the lysophospholipase screening plate added with uracil, which had its own lysophospholipase (LPL) enzyme activity increased by 10.8 times. Recombinant expression of the lysophospholipase LPL was carried out in it, and the expression capability was found to be significantly improved, which was increased by 112% compared with the starting strain. This strain can be used to heterologously express various proteins, especially lysophospholipases for food efficiently.

Specifically, the present invention relates to the following aspects:
In one aspect, the invention relates to a lysophospholipase comprising the amino acid sequence of SEQ ID NO: 14 or 16.

In another aspect, the present invention relates to a nucleic acid molecule comprising: (a) a nucleotide sequence encoding the above-mentioned lysophospholipase; and (b) a nucleotide sequence complementary to the nucleotide sequence described in (a), either partially or fully.

The skilled in the art will recognize that due to the degeneracy of the genetic codes, multiple different nucleotide sequences can encode the same enzyme. In addition, it will be recognized that the skilled in the art can use routine techniques to make nucleotide substitutions that do not affect the activity of the enzyme encoded by the nucleotide sequence of the invention and thus reflect the codon bias of any particular host organism in which the enzyme of the invention is expressed.

The invention also provides a vector comprising the nucleic acid molecule, and a host cell comprising the nucleic acid molecule or the vector.

"Vector" refers to an extrachromosomal element that usually carries a gene that is not a part of the central metabolism of a cell, and is often in the form of a circular double-stranded DNA molecule. Such elements may be autonomously replicating sequences, genomic integration sequences, phage or nucleotide sequences, linear or circular single- or double-stranded DNAs or RNAs from any source, in which many of the nucleotide sequences have been spliced or recombined into specific constructs that are capable of introducing the promoter fragments and DNA sequences of the selected gene products together with appropriate 3' untranslated sequences into a cell.

Genes and gene products encoding the lysophospholipases of the invention can be expressed in heterologous host cells, such as bacterial cells, fungal cells, such as yeast cells, mammalian cells, insect cells and plant cells. Heterologous host cells for expressing the nucleic acid molecules of the invention can be microbial hosts in fungal or bacterial families and grow over a wide range of temperature, pH, and solvent tolerance. For example, it is contemplated that any bacteria, yeast, and filamentous fungi may be suitable hosts for expression of the nucleic acid molecules of the invention. Examples of host strains include, but are not limited to, bacterial, fungal or yeast species such as Pichia, Aspergillus, Trichoderma, Saccharomyces, Phaffia , Kluyveromyces, Yarrowia, Candida, Hansenula, Salmonella, Bacillus, Acinetobacter, Zymomonas, Agrobacterium, Erythrobacter, Chlorobium, Chromatium, Flavobacterium, Cytophaga, Rhodobacter, Rhodococcus, Streptomyces, Brevibacterium, Corynebacteria, Mycobacterium, Deinococcus, Escherichia, Erwinia, Pantoea, Pseudomonas, Sphingomonas, Methylomonas, Methylobacter, Methylococcus, Methylosinus, Methylomicrobium, Methylocystis, Alcaligenes, Synechocystis, Synechococcus, Anabaena, Thiobacillus, Methanobacterium, Klebsiella and Myxococcus species. In one embodiment, the host cell is a fungal cell. In one embodiment, the host cell is a *Pichia pastoris* or *Aspergillus niger* cell. In one embodiment, the host cell is an *Aspergillus niger* cell of the present invention having a deposit number of CGMCC No. 40011.

Vectors useful for transforming the host cells described above are well known in the art. Typically, vectors contain sequences that direct transcription and translation of the related genes, selectable markers, and sequences that allow autonomous replication or chromosomal integration. A suitable vector contains a 5' region of the gene that controls transcription initiation and a 3' region of the DNA fragment that controls transcription termination.

In one aspect, the invention also relates to a method for producing a lysophospholipase, comprising expressing in a host cell a nucleic acid molecule encoding the lysophospholipase of the invention and recovering the resulting polypeptide.

A variety of culture methods can be used to prepare the enzymes of the invention. For example, large-scale production of specific gene products from recombinant microbial hosts can be performed by batch, fed-batch, and continuous culture methods.

Batch and fed-batch culture methods are commonly used and well known in the art, and examples can be found in: Thomas D. Brock in Biotechnology: A Textbook of Industrial Microbiology, Second Edition, Sinauer Associates, Inc., Sunderland, MA (1989)), and Deshpande, Mukund V., (Appl. Biochem. Biotechnol., 36: 227-234 (1992).

Commercial production of the enzymes of the invention can also be carried out by continuous culture. Continuous culture is an open system in which conditioned media are continuously added to the bioreactor and equal amounts of conditioned media are simultaneously removed for processing. Continuous culture generally maintains cells at a constant high liquid density where the cells are primarily in the logarithmic phase of growth. Alternatively, continuous culture can be performed with immobilized cells, wherein carbon and nutrients are continuously added and valuable products, byproducts or waste products are continuously removed from the cell pellet. Cell immobilization can be performed using a wide range of solid supports consisting of natural and/or synthetic materials.

Recovery of the desired enzyme from batch fermentation, fed-batch fermentation, or continuous culture can be accomplished by any method known to those skilled in the art. For example, when an enzyme is produced intracellularly, the cell slurry is separated from the culture medium by centrifugation or membrane filtration, optionally washed with water or an aqueous buffer of the desired pH. Then the cell slurry in the aqueous buffer of the desired pH is suspended to be homogenized to produce a cell extract containing the required enzyme.

The present invention also relates to a composition comprising the lysophospholipase of the invention, or a fermentation broth, fermentation supernatant and/or fermentation concentrate of the host cell of the invention. The enzyme composition of the present invention may be in any form suitable for use, for example, crude fermentation broth with or without cell removal, cell lysate with or without cell debris, semi-purified or purified enzyme composition, or host cells as a source of enzymes. The enzyme composition may be a dry powder or granule, a dust-free granule, a liquid, a stabilized liquid or a stabilized protected enzyme. The liquid enzyme composition can be stabilized according to established processes, for example by adding stabilizers such as sugars, sugar alcohols or other polyols, and/or lactic acid or other organic acids.

The present invention further relates to a fermentation broth, fermentation supernatant or fermentation concentrate of the host cell of the present invention.

The present invention also relates to use of the lysophospholipase of the present invention in oil degumming. When the lysophospholipase of the present invention is used for oil degumming, the specific enzyme activity is significantly improved compared to the lysophospholipase of the prior art, and the production cost is reduced.

In another aspect, the present invention relates to a mutated *Aspergillus niger* strain, which is an orotate phosphoribosyltransferase auxotrophic strain, and which has an increased production capacity of an endogenous enzyme, such a lysophospholipase relative to an unmutated strain. For example, it can be increased by 10-15 times, such as 10-12 times, especially 10.8 times.

In the present invention, the term "endogenous enzyme" refers to an enzyme expressed by the mutant *Aspergillus niger* strain itself, for example, including but not limited to a lysophospholipase expressed by the mutant *Aspergillus niger* strain itself.

In one embodiment, the orotate phosphoribosyltransferase pyrE gene of the mutated *Aspergillus niger* of the invention has a deletion of nucleotides TT at positions 64 and 65.

In one embodiment, the mutated *Aspergillus niger* strain of the invention has a deposit number of CGMCC No. 40011.

In addition to its own endogenous enzymes, the mutated *Aspergillus niger* strain of the present invention is capable of efficiently expressing heterologous proteins. The mutated *Aspergillus niger* strain of the present invention can be used to express a wide range of heterologous proteins, such as amylase, glucose oxidase, catalase, cellulase, pectinase, protease, phytase, xylanase, lysozyme, interleukin-6, human lactoferrin, bovine chymosin, thaumatin, lipase, etc. In particular, when the mutated *Aspergillus niger* strain of the present invention recombinantly expresses the lysophospholipase LPL, the protein expression level can be increased by more than 100% compared with the starting strain, for example, increased by 100%-150%, particularly 100%-120%, more particularly 100%-112%. Therefore, this strain has obvious utility for efficient expression of heterologous proteins.

In another aspect, the present invention relates to a recombinant *Aspergillus niger* strain obtained by introducing a gene encoding a foreign protein into the above-described mutated *Aspergillus niger* strain. In one embodiment, the exogenous proteins are enzymes and other proteins such as amylase, glucose oxidase, catalase, cellulase, pectinase, protease, phytase, xylanase, lysozyme, interleukin-6, human lactoferrin, bovine chymosin, thaumatin, lipase, etc., especially lysophospholipase.

In another aspect, the present invention relates to a method for producing a target protein, comprising introducing a gene encoding the target protein into the above-mentioned mutant *Aspergillus niger* strain, and culturing the strain to produce the target protein. Alternatively, the method comprises culturing the above recombinant *Aspergillus niger* strain to produce the target protein. In one embodiment, the exogenous proteins are enzymes and other proteins such as amylase, glucose oxidase, catalase, cellulase, pectinase, protease, phytase, xylanase, lysozyme, interleukin-6, human lactoferrin, bovine chymosin, thaumatin, lipase, etc., especially lysophospholipase.

In another aspect, the present invention relates to a biocatalyst, which comprises the mutated *Aspergillus niger* strain as described above into which a gene encoding a foreign protein is introduced. The foreign proteins are enzymes and other proteins, such as amylase, glucose oxidase, catalase, cellulase, pectinase, protease, phytase, xylanase, lysozyme, interleukin-6, human lactoferrin, bovine chymosin, thaumatin, lipase, etc., especially lysophospholipase.

In another aspect, the invention relates to a foreign protein produced by the strain described above. The foreign proteins are enzymes and other proteins such as amylase, glucose oxidase, catalase, cellulase, pectinase, protease, phytase, xylanase, lysozyme, interleukin- 6, human lactoferrin, bovine chymosin, thaumatin, lipase, etc., especially lysophospholipase. The foreign proteins can be used in food, preferably as an enzyme for food, more preferably as a lysophospholipase for food.

In one embodiment, the lysophospholipase is a mutated lysophospholipase of the invention.

The present invention also relates to a recombinant microbial cell into which an intrabacterial component derived from the above-described mutated *Aspergillus niger* strains is introduced. After obtaining the strain of the present invention, its intrabacterial components can be isolated by conventional techniques and introduced into other microorganisms. The recombinant microbial cells into which the components are introduced have the excellent properties of the strain of the present invention. In the present invention, the term "intrabacterial component" refers to the sum of all genetic materials of an organism, specifically including but not limited to: encoding DNAs, non-encoding DNAs, and mitochondrial DNAs.

In particular, the mutated *Aspergillus niger* strain of the present invention can be used to express foreign proteins. After the mutated *Aspergillus niger* of the present invention is obtained, a commonly used expression vector can be introduced into it for expressing foreign proteins. For example, the expression vector can contain a promoter and a terminator, with a multiple cloning site between the promoter and the terminator, where a gene encoding a foreign protein can be inserted. A promoter may comprise one or more copies of an enhancer. Many commercial vectors can be used to express foreign proteins in the mutated *Aspergillus niger* strain of the present invention.

When expressing foreign proteins derived from non-*Aspergillus niger* origins, the codons of some species may be rare ones in *Aspergillus niger.* Therefore, when introducing the expression vector, the gene encoding the foreign protein can be firstly codon-optimized for the *Aspergillus niger* of the present invention, thereby increasing the expression.

The *Aspergillus niger* of the present invention can express a variety of foreign proteins, including food enzymes, such a lipases for food, pharmaceutical proteins, various enzymes from plants, animals and bacteria, membrane receptor proteins, proteins comprising prosthetic groups, and proteins which can be used to study crystal structures, etc. The *Aspergillus niger* of the present invention which expresses foreign enzyme components can also be used as a biocatalyst via the whole cell.

In one embodiment, the mutated *Aspergillus niger* strain of the invention, for example an *Aspergillus niger* strain having the deposit number of CGMCC No. 40011, can be used to express a mutated lysophospholipase of the invention, for example the lysophospholipase comprising an amino acid sequence of SEQ ID NO: 14 or 16. Due to the combination effects of the two, the expression efficiency of the lysophospholipase is expected to be further improved.

### Brief description of drawings

Figure 1 shows specific enzyme activities directed against 1-palmitoyl lysophosphatidylcholine of the lysophospholipase LPLs encoded by picAN02m1 (M1) and pic-AN02-LPL (WT) of CN2021107079194, and the lysophospholipases of mutants W, Y, and F of the present invention.
Figure 2 is a schematic diagram of the LPL gene expression vector constructed in the present invention.
Figure 3 is a diagram of AN19E complementation and transformation plate.
Figure 4 is a diagram of the plate for screening the lysophospholipase LPL activity of AN19E complementation transformants.
Figure 5 is a diagram of the mutagenized strain AN19E-13 in LPL screening medium.
Figure 6 is a comparison chart of the enzyme activities of the endogenous lysophospholipase LPLs expressed by the mutated strain AN19E-13 of the present invention and the starting strain AN19E.
Figure 7 is a comparison chart of the enzyme activities of the lysophospholipase LPLs recombinantly expressed by the mutated strain AN19E-13 of the present invention and the starting strains CICC2243 and AN19E.
Figure 8 is a protein electrophoresis diagram of the lysophospholipase LPLs recombinantly expressed by the mutated strain AN19E-13 of the present invention and the starting strains CICC2243 and AN19E.

### Description of the deposit

The strain AN19E-13 of the present invention was deposited in China General Microbiological Culture Collection Center (CGMCC), Institute of Microbiology, Chinese Academy of Sciences, No. 1, West Beichen Road, Chaoyang District, Beijing, China, on December 20, 2021, with the deposit number CGMCC No. 40011, classified as *Aspergillus niger.*

### Detailed description of the invention

I. Preparation and functional verification of the mutated lysophospholipase of the present invention.

### Experimental Materials

*Aspergillus niger* strain GIM 3.24 (AN02) was purchased from Guangdong Microbial Culture Collection Center.

The formula of *Aspergillus niger* fermentation medium was:
2% glucose, 10% maltose, 7% sodium citrate, 1.5% ammonium sulfate, 4% Tryptic soy broth, 0.1% sodium dihydrogen phosphate, 0.1% magnesium sulfate, 0.07% Tween 80, trace elements (KI 0.83g/L, H₃BO₃ 6.2g/L, MnSO₄•4H₂O 22.3g/L, ZnSO₄•7H₂O 8.6g/L, Na₂MoO₄•2H₂O 0.25g/L, CuSO₄•5H₂O 0.025g/L, CoCl₂•6H₂O 0.025g/L added at the ratio of 1/1000, FeSO₄•7H₂O 2.78g/L, Na₂•EDTA 3.73g/L added at the ratio of 1/100).

The formula of lysis buffer was: 100mM Tris-HCl pH 8.0; 50mM Na•EDTA; 1% SDS.

BMMY-soybean lecithin medium:
Component A, BMMY solid medium: 1% yeast extract, 2% peptone, 100mM citric acid-sodium citrate buffer, pH 6.6, 1.34% YNB, 4 × 10-5% biotin (add before inverting the plate), 2% methanol (added before inverting the plate), 2% agar dissolved in 250 ml deionized water.

Component B, 250 ml of soybean lecithin substrate solution: 4% soybean lecithin, emulsified for 3 minutes with a high-speed homogenizer at 8000 rpm, paused for 1 minute and then emulsified for 3 minutes to prepare a substrate solution.

After sterilization, components A and B were mixed, 10 ml of methanol was added, and the plate was inverted.

Glass beads were purchased from Biospec, U.S.A. PrimeSTAR^{®}HS DNA Polymerase was purchased from Takara, Cat. No. R010A.

SalI, EcoRI and BglII restriction enzymes were purchased from NEB.

Lysophospholipid 1-palmitoyl-sn-glycero-3-phosphocholine was purchased from Aladdin, Cat. No. P130493-500 mg.

NEFA kit was purchased from Wako Pure Chemical Industries, Ltd., Japan.

Bradford kit was purchased from Sangon (Shanghai) Bioengineering Co., Ltd.

MGYS plate: 1.34% YNB, 4x 10-5% biotin (filter sterilized), 2% glycerol, 1M sorbitol.

YPD medium: 1% yeast extract, 2% peptone, 2% glucose.

BMGY shake flask medium: 1% yeast extract, 2% peptone, 100mM citric acid-sodium citrate buffer, pH 6.6, 1.34% YNB, 4x 10-5% biotin (filter sterilized), 2% glycerol.

BMMY shake flask medium: 1% yeast extract, 2% peptone, 100mM citric acid-sodium citrate buffer, pH 6.6, 1.34% YNB, 4x 10-5% biotin (filter sterilized), 2% methanol (added during inoculation).

### Example 1: cloning of Aspergillus niger lipaseB gene

*Aspergillus niger* strain GIM 3.24 (AN02) was cultured in *Aspergillus niger* fermentation medium at 30°C for 24 hours. The fermentation culture was centrifuged at 4000 rpm for 5 minutes, and the cells were taken out and suspended in 700ul lysis buffer, transferred to a cryovial, added with 300ul glass beads, vibrated on mini beadbeater for 40s, and centrifuged at 12000rpm for 10min. 600ul of the supernatant was taken out and added with 275ul of 7M ammonium acetate, and then was subjected to water bath at 65°C for 10 minutes and ice bath for 5 minutes. After equal volume of phenol, chloroform, isoamyl alcohol (24:25:1) was added, it was vortexed thoroughly, and centrifuged at 12000rpm for 5 minutes. The supernatant was taken and added with an equal volume of chloroform, vortexed thoroughly, and centrifuged at 12000rpm for 5min. The supernatant was taken and added with 2 volumes of absolute ethanol, placed at -80°C for 20min, centrifuged at 12000rpm for 10min to obtain a white DNA precipitate, which was washed twice with 70% ethanol. After the ethanol was fully evaporated, sterile water was added to dissolve the DNA.

Primers LPL-1 and LPL-2 were designed according to the gene sequence of lipaseB of *Aspergillus niger* CBS513.88 in NCBI.
LPL-1 5'-atgtttctccgcagggaatt-3' (SEQ ID NO: 1)
LPL-2 5'-ctacgagcattcactaatgt-3' (SEQ ID NO: 2).

The LPL DNA of *Aspergillus niger* strain GIM 3.24 (AN02) was cloned with PrimeSTAR^{®}HS DNA Polymerase. After TA cloning with Mighty TA-cloning Reagent Set for PrimeSTAR^{®} kit, it was transform into DH5a E. coli. The DNA sequencing was performed by Sangon Bioengineering Co., Ltd. The following LPL DNA sequence of GIM 3.24 (AN02) was obtained:
DNA sequence of GIM 3.24 (AN02) LPL:

atgtttctccgcagggaatttggggctgttgcagcCCTATCTGTGCTGGCCCATGCTGCTCCCGC
ACCTGCTCCGATGCAGCGTAGAGGTAAGACACACTTACCAATTTGCAGAAC
ACCCGCTAACCTACTCAGACATCTCCTCTACCGTCTTGGACAATATCGACCT
CTTCGCCCAATACAGTGCAGCAGCTTACTGCTCCTCGAACATCGAGTCCAC
CGGCACGACTCTGACCTGCGACGTAGGCAATTGCCCTCTCGTCGAGGCAG
CCGGTGCCACGACCATCGATGAGTTTGACGAGTAAGCCAATCCAACCCCAA
CATCTTCCCCCACTTGGCATCCAGCTCACACCCCCATAGCACCAGCAGCTA
CGGCGACCCGACTGGGTTCATCGCCGTTGACCCAACGAACGAGTTAATTGT
TCTGTCTTTCCGGGGTAGTTCCGACCTCTCGAACTGGATTGCCGACCTAGA
CTTCGGCCTCACCTCCGTAAGCAGCATCTGTGATGGCTGTGAGATGCACAA
GGGCTTCTACGAGGCCTGGGAAGTCATCGCGGACACCATCACTAGCAAGG
TGGAGGCTGCTGTCTCCAGCTATCCGGACTACACCCTCGTGTTCACTGGAC
ACAGCTACGGCGCTGCATTGGCGGCTGTCGCGGCCACCGTGCTCCGCAAC
GCCGGATACACTCTTGACCTGGTAAGTTCCTACTCTTTTATCCTTGTAACGT
TCCCCCATCATTCGGATGGTCTACTAACACAATCAACAGTACAACTTCGGCC
AGCCCCGTATTGGCAACCTCGCCTTAGCCGACTATATCACCGGCCAAAATAT
GGGCAGCAACTACCGCGTCACGCACACCGATGACATCGTGCCTAAGCTGC
CTCCGGAGCTGCTGGGCTACCACCACTTCAGCCCGGAGTACTGGATCACCA
GCGGTAATGATGTGACGGTGACTACGTCGGACGTGACCGAGGTCGTGGGG
GTGGATTCGACGGCTGGGAATGACGGCACGCTGCTTGACAGTACGACTGC
CCATCggtggtacacgatctacattagtgaatgctcgtag (SEQ ID NO:3).

According to the intron sequence of the DNA sequence of lipaseB of CBS513.88, the intron sequence of GIM 3.24 (AN02) was found and removed, and the DNA sequence was translated into an amino acid sequence. The results were as follows:
The amino acid sequence of GIM 3.24(AN02) LPL was as follows:

MFLRREFGAVAALSVLAHAAPAPAPMQRRDISSTVLDNIDLFAQYSAAAYCSS
NIESTGTTLTCDVGNCPLVEAAGATTIDEFDDTSSYGDPTGFIAVDPTNELIVLS
FRGSSDLSNWIADLDFGLTSVSSICDGCEMHKGFYEAWEVIADTITSKVEAAV

SSYPDYTLVFTGHSYGAALAAVAATVLRNAGYTLDLYNFGQPRIGNLALADYI
TGQNMGSNYRVTHTDDIVPKLPPELLGYHHFSPEYWITSGNDVTVTTSDVTE
VVGVDSTAGNDGTLLDSTTAHRWYTIYISECS (SEQ ID NO:4).

### Example 2: Expression and random mutation of AN02-LPL in Pichia pastoris

The mature peptide of AN02-LPL was selected, and the first 10 amino acids thereof was used as the leading peptide to obtain AN02-LPL. The amino acid sequence was as follows:

APAPAPMQRRDISSTVLDNIDLFAQYSAAAYCSSNIESTGTTLTCDVGNCPLVE
AAGATTIDEFDDTSSYGDPTGFIAVDPTNELIVLSFRGSSDLSNWIADLDFGLTS
VSSICDGCEMHKGFYEAWEVIADTITSKVEAAVSSYPDYTLVFTGHSYGAALA
AVAATVLRNAGYTLDLYNFGQPRIGNLALADYITGQNMGSNYRVTHTDDIVP
KLPPELLGYHHFSPEYWITSGNDVTVTTSDVTEVVGVDSTAGNDGTLLDSTTA
HRWYTIYISECS(SEQ ID NO:5).

The corresponding DNA sequence was as follows:

GCTCCAGCTCCTGCACCAATGCAAAGAAGAGATATTTCTTCTACCGTTTTG
GATAACATTGATTTGTTCGCTCAATACTCCGCTGCTGCTTACTGTTCTTCTAA
CATTGAGTCTACTGGTACCACTTTGACTTGTGACGTTGGTAACTGTCCATTG
GTTGAGGCTGCTGGTGCTACCACTATTGATGAGTTCGATGATACTTCCTCTT
ACGGTGATCCTACTGGTTTCATTGCTGTTGACCCTACTAACGAGTTGATTGT
TTTGTCTTTTAGAGGTTCTTCTGACTTGTCTAACTGGATTGCTGATTTGGATT
TCGGTTTGACTTCTGTTTCTTCTATTTGTGACGGTTGTGAAATGCACAAGGG
TTTCTATGAAGCCTGGGAAGTTATTGCTGATACTATTACTTCTAAGGTTGAA
GCTGCTGTTTCTTCTTACCCAGATTACACTTTGGTTTTCACCGGTCACTCTT
ACGGTGCTGCCTTGGCTGCTGTTGCTGCTACTGTTTTGAGAAACGCTGGTT
ACACTTTGGATTTGTACAACTTTGGTCAACCAAGAATTGGTAACTTGGCTTT
GGCTGACTACATTACTGGTCAAAACATGGGTTCTAACTACAGAGTTACTCA
CACTGATGATATTGTTCCTAAGTTGCCACCAGAGTTGTTGGGTTACCACCAC
TTCTCCCCAGAGTACTGGATTACTTCTGGTAACGATGTTACTGTTACCACTT
CTGATGTTACTGAGGTTGTTGGTGTTGATTCCACCGCTGGTAACGATGGTAC
TTTGTTGGACTCTACTACTGCTCACAGATGGTACACCATTTACATTTCTGAG
TGTTCTtaa (SEQ ID NO:6).

After a Sac-II restriction site CCGCGG was added to the 5' end, the gene sequence was synthesized by Sangon Bioengineering Co., Ltd, and was cloned into the pAO815 vector (purchased from invitrogen) through the EcoRI restriction site to obtain the pic-AN02 plasmid. After linearization with BglII, the vector was transformed into competent cells of *Pichia pastoris* GS115 strain (purchased from invitrogen) using electroporation. The transformants were inoculated on the MGYS plate and cultured at 30°C for 3 days to obtain *Pichia pastoris* transformants. Single clones on the plate were picked and placed on the BMMY-soybean phospholipid medium screening plate. A clone with a large white sedimentation circle was selected and named as pic-AN02-LPL.

Using pic-AN02-LPL as a template, error-prone PCR was performed with TaKaRa Taq enzyme and primer pair PLPL-1: TCCCCGCGGCGAAACGATGAGATTTCCTTC (SEQ ID NO: 7)/PLPL-2: CCGGAATTCTTAAGAACACTCAGAAATG (SEQ ID NO: 8) (0.3mM MnCl₂ was additionally added during PCR) to obtain a collection of mutant amplicon fragments with a size of approximately 1000 bp. The obtained fragment was cloned into pic-AN02 plasmid through Sac-II and EcoRI restriction sites, and the obtained vector was transformed into E. coli DH5α strain.

The plate containing the pic-AN02 mutant was washed with 2 ml of sterile water, and the plasmid was extracted, linearized with SalI, and a fragment of approximately 8.5 kb was recovered and used as the vector. 500ng of the vector was taken and transformed into competent cells of *Pichia pastoris* GS115 strain using electrotransformation method. The transformants were inoculated on the BMM-soybean phospholipid screening medium plate and cultured at 30°C for 3 days to obtain a *Pichia pastoris* mutant library of pic-AN02-LPL. A mutant strain pic-AN02m1 was obtained through screening.

The pic-AN02m1 strain was inoculated into 3 ml YPD liquid medium, cultured at 30°C overnight, and genomic DNA was extracted. Using the genomic DNA of the pic-AN0m1 strain as a template, PCR amplification was performed with PrimeSTAR^{®}HS DNA polymerase and primer pair AOX-5: GACTGGTTCCAATTGACAAGC (SEQ ID NO: 9) and 3'-AOX1: GGCAAATGGCATTCTGACATCCTC (SEQ ID NO: 10) to obtain the DNA sequence of the AN02 mutant in the pic-AN02m1 strain. The obtained sequence was sent to Shanghai Sangon Bioengineering Company to be sequenced with the primer pair AOX-5/3'-AOX1. The DNA sequence of the mutated LPL of the pic-AN02m1 strain and the amino acid encoded by it were as follows, and the mutation sites were L86I, G187D, E209K, and A245D.
Amino acid sequence: 
APAPAPMQRRDISSTVLDNIDLFAQYSAAAYCSSNIESTGTTLTCDVGNCPLVE
AAGATTIDEFDDTSSYGDPTGFIAVDPTNELIVLSFRGSSDISNWIADLDFGLTS
VSSICDGCEMHKGFYEAWEVIADTITSKVEAAVSSYPDYTLVFTGHSYGAALA
AVAATVLRNAGYTLDLYNFGQPRIGNLALADYITDQNMGSNYRVTHTDDIVP
KLPPKLLGYHHFSPEYWITSGNDVTVTTSDVTEVVGVDSTDGNDGTLLDSTT
AHRWYTIYISECS (SEQ ID NO:11)。
DNA sequence:

GCTCCAGCTCCTGCACCAATGCAAAGAAGAGATATTTCTTCTACCGTTTTG
GATAACATTGATTTGTTCGCTCAATACTCCGCTGCTGCTTACTGTTCTTCTAA
CATTGAGTCTACTGGTACCACTTTGACTTGTGACGTTGGTAACTGTCCATTG
GTTGAGGCTGCTGGTGCTACCACTATTGATGAGTTCGATGATACTTCCTCTT
ACGGTGATCCTACTGGTTTCATTGCTGTTGACCCTACTAACGAGTTGATTGT
TTTGTCTTTTAGAGGTTCTTCTGACATCTCTAACTGGATTGCTGATTTGGATT
TCGGTTTGACTTCTGTTTCTTCTATTTGTGACGGTTGTGAAATGCACAAGGG
TTTCTATGAAGCCTGGGAAGTTATTGCTGATACTATTACTTCTAAGGTTGAA
GCTGCTGTTTCTTCTTACCCAGATTACACTTTGGTTTTCACCGGTCACTCTT
ACGGTGCTGCCTTGGCTGCTGTTGCTGCTACTGTTTTGAGAAACGCTGGTT
ACACTTTGGATTTGTACAACTTTGGTCAACCAAGAATTGGTAACTTGGCTTT
GGCTGACTACATTACTGATCAAAACATGGGTTCTAACTACAGAGTTACTCAC
ACTGATGATATTGTTCCTAAGTTGCCACCAAAGTTGTTGGGTTACCACCACT
TCTCCCCAGAGTACTGGATTACTTCTGGTAACGATGTTACTGTTACCACTTC
TGATGTTACTGAGGTTGTTGGTGTTGATTCCACCGATGGTAACGATGGTACT
TTGTTGGACTCTACTACTGCTCACAGATGGTACACCATTTACATTTCTGAGT
GTTCTtaa (SEQ ID NO:12).

### Example 3: Site-directed mutagenesis of the LPL gene encoded by pic-AN02m1 in Pichia pastoris

The threonine at position 255 of the lysophospholipase LPL encoded by pic-AN02m1 was mutated to tryptophan, phenylalanine and tyrosine respectively, and the mutated genes were named W, F and Y.

Amino acid sequence of mutant W

APAPAPMQRRDISSTVLDNIDLFAQYSAAAYCSSNIESTGTTLTCDVGNCPLVE
AAGATTIDEFDDTSSYGDPTGFIAVDPTNELIVLSFRGSSDISNWIADLDFGLTS
VSSICDGCEMHKGFYEAWEVIADTITSKVEAAVSSYPDYTLVFTGHSYGAALA
AVAATVLRNAGYTLDLYNFGQPRIGNLALADYITDQNMGSNYRVTHTDDIVP
KLPPKLLGYHHFSPEYWITSGNDVTVTTSDVTEVVGVDSTDGNDGTLLDSwT
AHRWYTIYISECS (SEQ ID NO:13).

The first ten amino acids thereof were the leader peptide. Therefore, its functional sequence was as follows:

DISSTVLDNIDLFAQYSAAAYCSSNIESTGTTLTCDVGNCPLVEAAGATTIDEFD
DTSSYGDPTGFIAVDPTNELIVLSFRGSSDISNWIADLDFGLTSVSSICDGCEMH
KGFYEAWEVIADTITSKVEAAVSSYPDYTLVFTGHSYGAALAAVAATVLRNAG
YTLDLYNFGQPRIGNLALADYITDQNMGSNYRVTHTDDIVPKLPPKLLGYHHF
SPEYWITSGNDVTVTTSDVTEVVGVDSTDGNDGTLLDSwTAHRWYTIYISECS
(SEQ ID NO:14).

Amino acid sequence of mutant F

APAPAPMQRRDISSTVLDNIDLFAQYSAAAYCSSNIESTGTTLTCDVGNCPLVE
AAGATTIDEFDDTSSYGDPTGFIAVDPTNELIVLSFRGSSDISNWIADLDFGLTS
VSSICDGCEMHKGFYEAWEVIADTITSKVEAAVSSYPDYTLVFTGHSYGAALA
AVAATVLRNAGYTLDLYNFGQPRIGNLALADYITDQNMGSNYRVTHTDDIVP
KLPPKLLGYHHFSPEYWITSGNDVTVTTSDVTEVVGVDSTDGNDGTLLDSFT
AHRWYTIYISECS (SEQ ID NO:15).

The first ten amino acids thereof were the leader peptide. Therefore, its functional sequence was as follows:

DISSTVLDNIDLFAQYSAAAYCSSNIESTGTTLTCDVGNCPLVEAAGATTIDEFD
DTSSYGDPTGFIAVDPTNELIVLSFRGSSDISNWIADLDFGLTSVSSICDGCEMH
KGFYEAWEVIADTITSKVEAAVSSYPDYTLVFTGHSYGAALAAVAATVLRNAG
YTLDLYNFGQPRIGNLALADYITDQNMGSNYRVTHTDDIVPKLPPKLLGYHHF
SPEYWITSGNDVTVTTSDVTEVVGVDSTDGNDGTLLDSFTAHRWYTIYISECS
(SEQ ID NO:16).

Amino acid sequence of mutant Y

APAPAPMQRRDISSTVLDNIDLFAQYSAAAYCSSNIESTGTTLTCDVGNCPLVE
AAGATTIDEFDDTSSYGDPTGFIAVDPTNELIVLSFRGSSDISNWIADLDFGLTS
VSSICDGCEMHKGFYEAWEVIADTITSKVEAAVSSYPDYTLVFTGHSYGAALA
AVAATVLRNAGYTLDLYNFGQPRIGNLALADYITDQNMGSNYRVTHTDDIVP
KLPPKLLGYHHFSPEYWITSGNDVTVTTSDVTEVVGVDSTDGNDGTLLDSYT
AHRWYTIYISECS (SEQ ID NO:17).

The first ten amino acids thereof were the leader peptide. Therefore, its functional sequence was as follows:

DISSTVLDNIDLFAQYSAAAYCSSNIESTGTTLTCDVGNCPLVEAAGATTIDEFD
DTSSYGDPTGFIAVDPTNELIVLSFRGSSDISNWIADLDFGLTSVSSICDGCEMH
KGFYEAWEVIADTITSKVEAAVSSYPDYTLVFTGHSYGAALAAVAATVLRNAG
YTLDLYNFGQPRIGNLALADYITDQNMGSNYRVTHTDDIVPKLPPKLLGYHHF
SPEYWITSGNDVTVTTSDVTEVVGVDSTDGNDGTLLDSYTAHRWYTIYISECS
(SEQ ID NO:18).

The gene sequences were synthesized by Sangon Bioengineering Co., Ltd. and were cloned into pic-AN02-LPL vector to obtain mutant W, F and Y plasmids respectively. After linearization with SalI, the vector was transformed into competent cells of *Pichia pastoris* GS115 strain using electroporation. The transformants were inoculated on the MGYS plate and cultured at 30°C for 3 days to obtain *P. pastoris* transformants of mutants W, F and Y. Single clones on the plate were picked and placed on the BMM-soybean phospholipid medium screening plate. Clones with large white sedimentation circles were selected and named as mutants W, F and Y respectively.

pic-AN02m1, pic-AN02-LPL *Pichia pastoris* expression strains and mutant W, F and Y *Pichia pastoris* expression strains were picked, firstly activated in liquid YPD, and then inoculated into BMGY medium, and cultured overnight at 30°C with shaking at 220 rpm. The culture was transferred to BMMY medium with an initial OD600 of 6.

Firstly, 2% methanol was used for induction. 1% methanol was added after 24h and 32h respectively. 1% methanol was added after 48h and 56h respectively, and sampling was done at 72h.

The fermentation broth obtained was subjected to ultrafiltration, desalination and concentration 30 times using an ultrafiltration tube with a molecular weight cutoff of 10 kDa. The processed sample was added into the buffer (20mM citric acid-sodium citrate buffer (pH 4.0)).

The lysophospholipase activity of the concentrated enzyme solution was determined as follows:
9ml substrate: 5ml 1% lysophospholipid, 1ml 20% Triton X-100, 2.5ml 0.1M citric acid-sodium citrate buffer.

10ul diluted enzyme solution + 90ul substrate were reacted at 50°C for 10 minutes, inactivated at 95°C for 5 minutes, and centrifuged at 7000rpm for 5 minutes. 1ul of the supernatant was taken and added with 80ul of reagent A in the NEFA kit, reacted at 37°C for 10 minutes, added with 160ul of reagent B and reacted for 10 minutes. The absorbance value at 550nm was determined to calculate the enzyme activity.

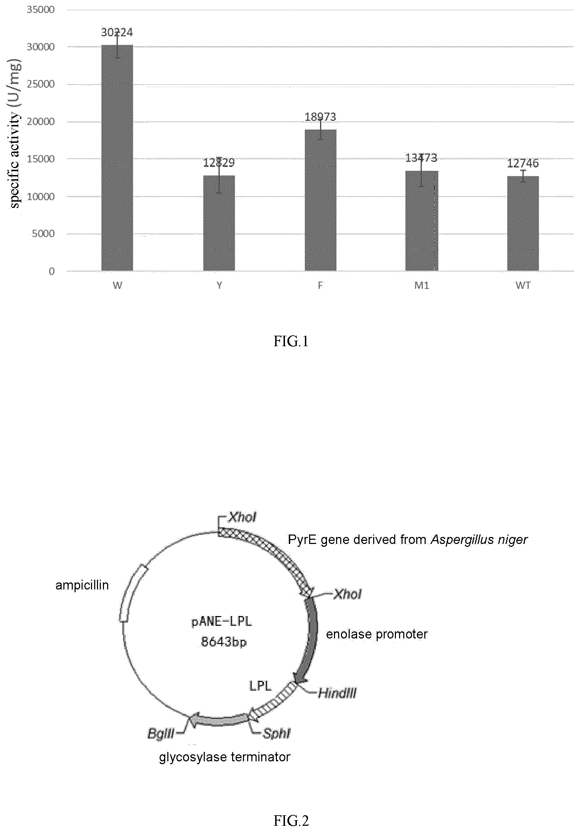

Protein concentration was determined using Bradford kit. The specific enzyme activities of LPL encoded by picAN02m1, LPL encoded by pic-AN02-LPL, and lysophospholipases of mutants W, Y, and F were calculated by calculating the enzyme activity and protein concentration. As shown in Figure 1, the specific enzyme activities of lysophospholipases of mutants W and F were 30224U/mg and 18973U/mg respectively; the specific enzyme activities of lysophospholipases of picAN02m1 (M1) and pic-AN02-LPL (WT) were 13473U/mg and 12746U/mg respectively. Compared with the starting sequence M1, that is, picAN02m1, which had the specific enzyme activity of 13473U/mg, the specific enzyme activities were increased by 124% and 40% respectively.

II. Preparation and functional verification of the mutated *Aspergillus niger* strain of the invention.

The starting *Aspergillus niger* strain was purchased from China Center of Industrial Culture Collection (CICC), with the deposit number of CICC2243. The starting strain was first spread on an MM solid medium plate to cultivate spores. After the spores were eluted, they were subjected to UV mutagenesis and finally were screened by adding 5-fluoroorotic acid and uracil to the screening plate to obtain the orotate phosphoribosyltransferase auxotrophic strain AN19E.

Then using ARTP mutagenesis, a strain AN19E-13 was screened. On the lysophospholipase screening plate added with uracil, the sedimentation circle of AN19E-13 was significantly larger than that of the starting strain AN19E. The test found that AN19E-13's own lysophospholipase (LPL) enzyme activity was 10.8 times higher than that of the starting strain AN19E.

An attempt was made to perform heterologous recombinant expression of lysophospholipase (LPL) in *Aspergillus niger* strain AN19E-13, and it was found that the ability of this strain to express LPL was increased by 112%. This proved that it had obvious advantages as a protein expression system, especially as an expression system for lysophospholipase used in food.

In the present invention, the term "ARTP" is the abbreviation of Atmospheric and Room Temperature Plasma, specifically refers to a plasma jet capable of producing high concentrations of active particles (including excited helium atoms, oxygen atoms, nitrogen atoms, OH radicals, etc.) at temperatures between 25-40 ° C under atmospheric pressure. The term "ARTP mutagenesis" refers to the use of normal pressure and room temperature plasma technology for strain mutagenesis. Specifically, the normal pressure and room temperature plasma source using helium as the working gas contains a variety of chemically active particle components, such as OH, nitrogen molecule two positive system, nitrogen molecule one negative system, excited state helium atom, hydrogen atom and oxygen atom, etc. The active energy particles rich in ARTP cause damage to the genetic materials of strains/plants/cells, etc., and induce biological cells to initiate the SOS repair mechanism. The SOS repair process is a highly error-tolerant repair process, so a rich variety of mismatch sites will be generated during the repair process, and ultimately the inheritance will be stabilized to form mutated strains. The strength of SOS repair is closely related to the degree of DNA damage.

### Example 4: Obtaining orotate phosphoribosyltransferase auxotrophic Aspergillus niger strain

Spores of *Aspergillus niger* CICC2243 strain were inoculated and coated onto MM solid medium (1% glucose, 0.5% KH₂PO₄, 0.6% NaNO₃, 0.05% KCl, 0.05% MgSO₄, 2% agar powder), and stationarily cultured at 28°C for 5 days to obtain *Aspergillus niger* spores. Fresh spores of *Aspergillus niger* CICC2243 strain were eluted with spore washing solution (0.9% NaCl, 0.05% Tween 80), and were filtered through Miracloth (Calbiochem, Cat#475885) to prepare a spore suspension. The cells were washed twice with sterile water and were adjusted to 1×10⁷ cells/mL. 2 mL of spore suspension was dispersed evenly on the surface of a petri dish, which was placed under the ultraviolet lamp of a clean workbench and irradiated for 90 seconds. 100 µL was taken and coated on MM solid medium added with 0.3% uracil and 1 mg/mL 5-fluoroorotic acid (5 -FOA), cultured at 28°C in the dark (the entire process was operated under red light to prevent reverse mutation) for 7 days. Single colonies grown on MM solid medium in the previous step were transferred to MM solid medium and MM-Uracil solid medium, and strains that could only grow on MM-Uracil solid medium were selected to obtain the orotate phosphoribosyltransferase auxotrophic strain AN19E. By sequencing the pyrE gene of *Aspergillus niger* AN19E strain, it was found that the 64th and 65th nucleotides TT were deleted, resulting in the inactivation of the pyrE gene.

### Example 5: Construction of LPL expression vector

The sequence of the exogenous *Aspergillus niger* lysophospholipase (LPL) gene was as follows:
Nucleic acid sequence: 
gctcccgcacctgctccgatgcagcgtagaggtaagacacacttaccaatttgcagaacacccgctaacctactcagacat
ctcctctaccgtcttggacaatatcgacctcttcgcccaatacagtgcagcagcttactgctcctcgaacatcgagtccaccg
gcacgactctgacctgcgacgtaggcaattgccctctcgtcgaggcagccggtgccacgaccatcgatgagtttgacgag
taagccaatccaaccccaacatcttcccccacttggcatccagctcacacccccatagcaccagcagctacggcgacccg
actgggttcatcgccgttgacccaacgaacgagttaattgttctgtctttccggggtagttccgacctctcgaactggattgcc
gacctagacttcggcctcacctccgtaagcagcatctgtgatggctgtgagatgcacaagggcttctacgaggcctgggaa
gtcatcgcggacaccatcactagcaaggtggaggctgctgtctccagctatccggactacaccctcgtgttcactggacac
agctacggcgctgcattggcggctgtcgcggccaccgtgctccgcaacgccggatacactcttgacctggtaagttcctac
tcttttatccttgtaacgttcccccatcattcggatggtctactaacacaatcaacagtacaacttcggccagccccgtattggc
aacctcgccttagccgactatatcaccggccaaaatatgggcagcaactaccgcgtcacgcacaccgatgacatcgtgcct
aagctgcctccggagctgctgggctaccaccacttcagcccggagtactggatcaccagcggtaatgatgtgacggtgac
tacgtcggacgtgaccgaggtcgtgggggtggattcgacggctgggaatgacggcacgctgcttgacagtacgactgcc
catcggtggtacacgatctacattagtgaatgctcgtag (SEQ ID NO:19).
Amino acid sequence:

APAPAPMQRRDISSTVLDNIDLFAQYSAAAYCSSNIESTGTTLTCDVGNCPLVE
AAGATTIDEFDDTSSYGDPTGFIAVDPTNELIVLSFRGSSDLSNWIADLDFGLTS
VSSICDGCEMHKGFYEAWEVIADTITSKVEAAVSSYPDYTLVFTGHSYGAALA
AVAATVLRNAGYTLDLYNFGQPRIGNLALADYITGQNMGSNYRVTHTDDIVP
KLPPELLGYHHFSPEYWITSGNDVTVTTSDVTEVVGVDSTAGNDGTLLDSTTA
HRWYTIYISECS (SEQ ID NO:20).

The specific operations were according to the method of "Molecular Cloning Experimental Guide" (Third Edition, New York: Cold Spring Harbor Laboratory Press, 1989). The LPL gene expression vector pANE-LPL as shown in Figure 2 was constructed as follows:
The LPL gene (SEQ ID NO: 19, containing *Aspergillus oryzae* α-amylase signal peptide (NCBI Sequence Number: XM_001821384.2, 1-63bp sequence)) obtained from the whole gene synthesis by Sangon (Shanghai) Bioengineering Co., Ltd was inserted into an expression cassette comprising the *Aspergillus oryzae* enolase promoter (NCBI sequence number: D63941.1, 215-734bp; containing 12 copies of the enhancer sequence (gtcgtgtcgggcatttatcgggggatggaccaatcagcgtagg, SEQ ID NO: 21) ) and the *Aspergillus niger* glycosylase terminator (NCBI sequence number: AF214480.1, the terminator sequence part) via SphI and HindIII enzyme sites. The entire expression cassette was inserted into the multiple cloning site of the cloning vector pSP72 via BglII and XhoI, and finally the PyrE expression gene derived from *Aspergillus niger* (NCBI sequence number: AY840014.1) was inserted into the vector via the XhoI restriction site to construct the LPL gene expression vector pANE-LPL. See Figure 2.

### Example 6: Aspergillus niger AN19E complementation experiment

Fresh spores of *Aspergillus niger* AN19E were eluted with spore washing solution, filtered through Miracloth to prepare a spore suspension, and adjusted to 1×10⁷ spores/mL. 1 mL of spore suspension was inoculated into mycelia culture medium (2% tryptone, 1% yeast extract, 2% glucose, 0.3% uracil), cultured for 40 hours at 28°C, 180 rpm. Grown mycelia was collected by sterilized Miracloth filtration.

The collected mycelia were washed three times with sterilized osmotic pressure stabilizer (0.6M MgSO₄, 10mM NaH₂PO₄, pH=5.8) and pressed dry. The mycelia were transferred to a 100mL Erlenmeyer flask, and every 0.8g of mycelia were suspended in 20mL of enzymatic hydrolysis solution (an osmotic pressure stabilizer was used to prepare an enzymatic hydrolysis solution of 1% lytic enzyme, 1% cellulase, and 0.1% helicase, which was sterilized by 0.22 µm microporous membrane filter), and were subjected to enzymatic hydrolysis at 30°C, 90 rpm for 60-90 minutes. The enzymatically digested protoplast mixture was filtered with Miracloth. The filtrate was collected, centrifuged at 1000g for 10 minutes at 4°C. The protoplast pellet was re-suspended in 5 mL of pre-cooled 1.0mol/L sorbitol solution, centrifuged at 800g for 10 minutes at 4°C, and the supernatant was discarded. Then the protoplasts were adjusted to 1×10⁷/mL with pre-cooled STC solution (1.0M sorbitol, 50mM CaCl₂, 50mM Tris-HCl, pH=7.5), and kept in ice bath until use.

200 µL protoplast suspension was added with 10 µL LPL expression vector pANE-LPL at a concentration of 1 µg/µL, then added with 50 µL PTC solution (40% PEG4000, 50mM CaCl₂, 50mM Tris-HCl, pH=7.5), mixed well, and kept in ice bath for 30 minutes. Then, it was added with 0.2mL PTC solution, mixed well, then added with 0.8mL PTC solution, mixed well, and kept at room temperature for 30 minutes.

The above mixture was coated on the regeneration medium (1% glucose, 0.6% NaNO₃, 0.15% KH₂PO₄, 0.05% KCl, 0.05% MgSO₄, 0.001% FeSO₄, 1M sucrose, 2% agar powder), and cultured at 28°C for 7 days until the colonies grew.

The colonies grown on the plate were transferred to lysophospholipase LPL screening medium and cultured at 28°C for 3 days.

The components of LPL screening medium were as follows:
Solution A: 2% maltose, 1.34% YNB, citric acid 6.88g/500mL, sodium citrate 5.07g/500mL, 5mM CaCl₂, added with water to 200mL.
Solution B: 1% lecithin, added with 200ml of water, emulsified with homogenizer, 2% agarose, 0.02% Triton-x-100, added with water to 300ml

After sterilization, A and B solutions were mixed and the plate was inverted.

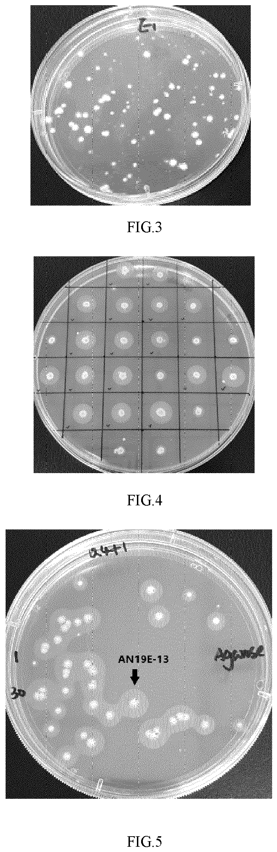

After transformation, it was found that *Aspergillus niger* AN19E could well achieve the pyrE gene complementation experiment, and the transformants showed lysophospholipase LPL activity in the lysophospholipase LPL screening medium, as shown in Figures 3 and 4, which further proved the successful establishment of the *Aspergillus niger* expression system which used the orotate phosphoribosyltransferase auxotrophic *Aspergillus niger* AN19E as the host.

### Example 7: Aspergillus niger AN19E ARTP mutagenesis experiment

Fresh spores of *Aspergillus niger* AN19E were eluted with spore washing solution, filtered through Miracloth to prepare a spore suspension, and adjusted to 2×10⁷ spores/mL. After mixing the spore suspension and 10% glycerol at 1:1, 10 µL of the mixed solution was taken onto an iron sheet and treated with an ARTP instrument (Wuxi Tmaxtree Biotechnology Co., Ltd., instrument model: ARTP-M) for 100 s. The instrument parameter setting was: radio frequency power range of 120W, helium volume of 10SLM (99.999% high-purity helium), and irradiation distance of 2mm.

After the treatment was completed, the iron sheet was removed and put into a centrifuge tube filled with 1 mL of sterile water, and then a pipette tip was used to repeatedly absorb to wash off the cells on the iron sheet. The cells were diluted to about 100 colonies/screening plate, and finally coated on the lysophospholipase screening plate added with uracil. The plate was then placed in a 30°C incubator for 3 days.

### Example 8: Screening of the mutagenized strains

The spores mutagenized in Example 7 were coated on a lysophospholipase screening plate added with uracil for screening. A strain with a significantly enlarged sedimentation circle was found (Figure 5), and was named as AN19E-13.

The strain AN19E-13 with significantly enlarged sedimentation circle and the starting strain AN19E were fermented in shake flasks added with the fermentation medium (2% maltose, 1.34% YNB, 1.38% citric acid, 1% sodium citrate, 5mM CaCl₂, 1% lecithin) autoclave sterilized at 115°C for 15min. The fermentation conditions were 28°C, 200rpm, 5d, inoculation volume of 1×10⁷ spores/50mL. Lysophospholipase LPL activity was determined.

The method for determining lysophospholipase LPL activity was as follows:
9mL substrate: 5mL 1% soy lecithin, 1mL 20% Triton X-100, 2.5mL 0.1M pH4.0 citric acid-sodium citrate buffer.

10uL appropriately diluted enzyme solution + 90uL substrate were reacted at 50°C for 10 minutes, inactivated at 95°C for 5 minutes, and centrifuged at 7000rpm for 5 minutes. 1uL supernatant was taken and added with 80uL of reagent A in the NEFA kit (Wako: 294-63601), reacted at 37°C for 10 minutes, added with 160uL of reagent B and reacted for 10 minutes. The absorbance value at 550nm was measured.

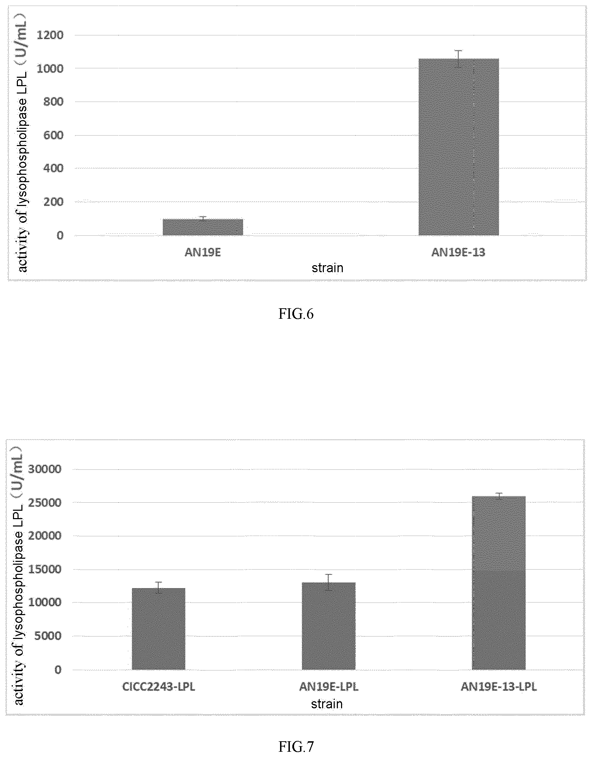

The results of enzyme activity measurement are shown in Figure 6.

The results showed that the activity of lysophospholipase LPL expressed by strain AN19E-13 with a significantly enlarged sedimentation circle was 1058 U/mL, which was 10.8 times higher than that of the starting strain AN19E (98 U/mL).

Strain AN19E-13 was deposited on December 20, 2021 at China General Microbiological Culture Collection Center (CGMCC), Institute of Microbiology, Chinese Academy of Sciences, No. 1, West Beichen Road, Chaoyang District, Beijing, with the deposit number of CGMCC No. 40011, classified as *Aspergillus niger.*

### Example 9: Investigation of the expression ability of Aspergillus niger AN19E-13

The transformation operation of *Aspergillus niger* AN19E-13 was the same as Example 6. The LPL expression vector pANE-LPL was transformed into the strain for recombinant expression.

The *Aspergillus niger* AN19E transformant of Example 6 was used as a control, and pANE-LPL was transformed into *Aspergillus niger* CICC2243. Since pANE-LPL could not use pyrE as the screening marker, p3SR2 (BCCM/LMBP: Deposit number: 2363) comprising acetamidase (amdS) gene was used for transformation screening. The regeneration medium needed to remove sodium nitrate and to add 15mM acetamide and 20mM cesium chloride.

For each of the three transformants, 40 transformants were selected for shaking flask fermentation. The fermentation medium (2% glucose, 15% maltose, 7% sodium citrate, 1.5% ammonium sulfate, 4% TSB, 0.1% dihydrogen phosphate sodium, 0.1% magnesium sulfate, 0.07% Tween 80, trace elements) was autoclave sterilized at 115°C for 15min. The fermentation conditions were: 28°C, 200rpm, 8d, inoculation amount of 1×10⁷ spores/50mL. The activity of lysophospholipase LPL was measured.

The method for measuring lysophospholipase LPL activity was shown in Example 8.

The results of enzyme activity measurement are shown in Figure 7.

The results showed that the enzyme activity of lysophospholipase LPL recombinantly expressed by AN19E-13 was 25920U/mL, which was increased by 112% and 100% respectively compared with the starting strains CICC2243 (12230U/mL) and AN19E (12990U/mL).

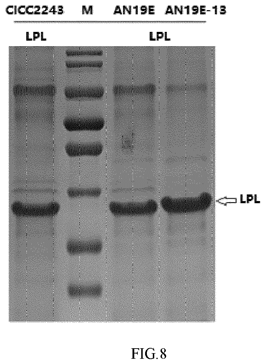

Polyacrylamide gel electrophoresis analysis: A 0.22 µm filter membrane was used to filter the supernatant. After an equal amount of the supernatant was concentrated to the same volume using a Milipore 10KDa ultrafiltration concentration tank, the same volume of concentrated enzyme solution was used for polyacrylamide gel electrophoresis analysis. The electrophoresis results are shown in Figure 8.

The results of protein electrophoresis showed that the concentration of LPL protein band of AN19E-13 transformant was significantly higher than that of the control.

## Claims

1. A lysophospholipase comprising the amino acid sequence of SEQ ID NO: 14 or 16.

2. A method for producing a lysophospholipase, comprising expressing in a host cell a nucleic acid molecule encoding the lysophospholipase of claim 1 and recovering the resulting polypeptide.

3. A mutated *Aspergillus niger* strain for expressing a lysophospholipase for food, which is an orotate phosphoribosyltransferase auxotrophic strain, and which has an increased production capacity of an endogenous enzyme, preferably lysophospholipase, relative to an unmutated strain.

4. The mutated *Aspergillus niger* strain of claim 3, which has a deletion of nucleotides TT at positions 64 and 65 of the orotate phosphoribosyltransferase pyrE gene thereof.

5. A mutated *Aspergillus niger* strain, which has a deposit number of CGMCC No. 40011.

6. A nucleic acid molecule comprising:
(a) a nucleotide sequence encoding the lysophospholipase of claim 1; and
(b) a nucleotide sequence complementary to the nucleotide sequence described in (a).

7. A vector comprising the nucleic acid molecule of claim 6.

8. A host cell comprising the nucleic acid molecule of claim 6 or the vector of claim 7.

9. The host cell of claim 8, wherein said host cell is selected from the group consisting of bacterial cells, fungal cells, mammalian cells, insect cells and plant cells.

10. The host cell of claim 9, wherein the host cell is a fungal cell, preferably a *Pichia pastoris* cell or an *Aspergillus niger* cell, preferably an *Aspergillus niger* cell having a deposit number of CGMCC NO.40011.

11. A composition comprising the lysophospholipase of claim 1, or the fermentation broth, fermentation supernatant and/or fermentation concentrate of the host cell of any one of claims 8-10.

12. A fermentation broth, fermentation supernatant or fermentation concentrate of the host cell of any one of claims 8-10.

13. Use of the lysophospholipase of claim 1 or the composition of claim 11 or the fermentation broth, fermentation supernatant and/or fermentation concentrate of the host cell of any one of claims 8-10 in oil degumming.

14. A recombinant *Aspergillus niger* strain obtained by introducing a gene encoding a foreign protein into the strain of any one of claims 3-5.

15. The recombinant *Aspergillus niger* strain of claim 14, wherein the foreign protein is an enzyme, preferably a lysophospholipase, preferably the lysophospholipase of claim 1.

16. A method for producing a target protein, comprising introducing a gene encoding the target protein into the strain of any one of claims 3-5, and culturing the strain to produce the target protein, or culturing the recombinant *Aspergillus niger* strain of any one of claims 14-15 to produce the target protein.

17. The method of claim 16, wherein the target protein is an enzyme, preferably a lysophospholipase, preferably the lysophospholipase of claim 1.

18. A biocatalyst, which comprises a mutated *Aspergillus niger* strain of any one of claims 3-5, into which a gene encoding an enzyme, preferably a lysophospholipase, preferably the lysophospholipase of claim 1 is introduced.

19. A foreign protein produced by the strain of any one of claims 3-5 and 14-15, wherein the foreign protein is an enzyme, preferably a lysophospholipase, and/or the foreign protein can be used in food, preferably as an enzyme for food, more preferably as a lysophospholipase for food.

20. A recombinant microbial cell into which an intrabacterial component derived from the strain of any one of claims 3-5 is introduced.
